Europäisches Patentamt

European Patent Office

Office européen des brevets

(11) Veröffentlichungsnummer: **0 037 488**
**B1**

# (12) EUROPÄISCHE PATENTSCHRIFT

(45) Veröffentlichungstag der Patentschrift:
**19.09.84**

(21) Anmeldenummer: **81102031.2**

(22) Anmeldetag: **19.03.81**

(51) Int. Cl.³: **A 61 K 31/70**, A 61 K 7/00,
A 61 K 9/00

(54) **Pharmazeutisches Mittel zur Heilung von entzündlichen und/oder degenerativen und/oder atrophischen Schleimhauterkrankungen.**

(30) Priorität: **25.03.80 DE 3011437**

(43) Veröffentlichungstag der Anmeldung:
**14.10.81 Patentblatt 81/41**

(45) Bekanntmachung des Hinweises auf die Patenterteilung:
**19.09.84 Patentblatt 84/38**

(84) Benannte Vertragsstaaten:
**AT BE CH DE FR GB IT LI LU NL SE**

(56) Entgegenhaltungen:
**FR - M - 88**

**THE MERCK INDEX, 9. Auflage, Merck & Co. Inc., 1976, Seite 657, Rahway, N.J., (USA)**

**Die Akte enthält technische Angaben, die nach dem Eingang der Anmeldung eingereicht wurden und die nicht in dieser Patentschrift enthalten sind.**

(73) Patentinhaber: **Krnjevic, Hrvoje, Dr., A. Santica 2/II, YU-41.000 Zagreb (YU)**

(72) Erfinder: **Krnjevic, Hrvoje, Dr., A. Santica 2/II, YU-41.000 Zagreb (YU)**

(74) Vertreter: **Ratzel, Gerhard, Dr., Seckenheimer Strasse 36a, D-6800 Mannheim 1 (DE)**

**Beschreibung**

Die Erfindung betrifft ein pharmazeutisches Mittel zur Heilung von entzündlichen und/oder degenerativen und/oder atrophischen Schleimhauterkrankungen, insbesondere bei topischer (lokaler) Anwendung, die durch den Gehalt an dem Nukleosid Inosin und an Lysinorotat gekennzeichnet sind.

Eine bevorzugte Ausführungsform dieses pharmazeutischen Mittels ist ferner durch einen Gehalt an Vitamin A und/oder Vitamin $B_{12}$ und/oder Folsäure gekennzeichnet.

Eine weitere bevorzugte Ausführungsform des erfindungsgemäßen Mittels ist durch einen Gehalt an Vitamin A und/oder an Thymin gekennzeichnet.

Das erfindungsgemäße pharmazeutische Mittel kann ferner einen an sich bekannten Salbengrundstoff, insbesondere einen an sich bekannten Hautsalbengrundstoff enthalten; es kann ferner in Gelform vorliegen.

Vorliegender Erfindung liegt insbesondere auch die überraschende Erkenntnis zugrunde, daß die beiden essentiellen Inhaltsstoffe Inosin und Lysinorotat eine gegenseitige synergistische Verstärkung bewirken. Dieser synergistische Effekt erscheint stark bei topischer (= lokaler) Anwendung.

Das neue erfindungsgemäße pharmazeutische Mittel ist insbesondere wirksam zur Heilung bzw. Verhinderung von Paradentose; Gingivitis (=Zahnfleischentzündungen); Stomatitis aphtosa (=Mundhöhlengeschwür); chronischer Conjunctivitis (=Bindehautentzündung); Gastritis; Ulcus cruris, Magengeschwür; Entzündungen im Vaginalbereich (z. B. Vaginitis); Fissuren und Raghaden, Beschädigungen an den Lippen des Mundes; Rhinitis und Hautverbrennungen.

Zum Stand der Technik ist zu sagen, daß gegen verschiedene der oben genannten Krankheiten bzw. pathologischen Zustände man bisher überhaupt noch keine sehr wirksamen Pharmazeutika bzw. Heilmittel kennt; beispielsweise ist dies bei Paradentose und bei degenerativer oder atrophischer Gingivitis der Fall.

Selbst gegen Gastritis hat man bisher noch kein echtes kausales Arzneimittel vorgeschlagen. Bei atrophischer und degenerativer Vaginitis helfen Antibiotika und andere an sich bekannte entzündungshemmende Arzneimittel, ferner auch das Breitband-Antibiotikum Oxytetracyclin wenig. Bei Rißfurchenbildung an den Lippen des Mundes helfen Antibiotika ebenfalls kaum.

Demgegenüber heilt das erfindungsgemäße pharmazeutische Mittel die vorgenannten pathologischen Zustände bzw. Krankheiten rasch und nachhaltig, zum Teil innerhalb einer Nacht, (z. B. bei Lippenriß, Blutungen bei Gingivitis bzw. Paradentose, Vaginitis, Rhinitis).

Zur Darlegung der therapeutischen Effekte wird folgendes ausgeführt:

Es wurde an insgesamt 580 Shay-Ratten die folgenden Versuche durchgeführt.

Die Versuche wurden an Wistar Ratten beiderlei Geschlechts vom Gewicht 150—220 g durchgeführt.

Die Magenulzera wurden nach einer modifizierten Shay-Methode hervorgerufen. Für 42 Stunden vor der Operation wurden die Ratten nüchtern gehalten. Sie wurden in Käfigen mit Gitterboden gehalten, zum Durchfallen der Fäkalien durch das Gitter, um zu verhindern, daß die Tiere eigene Fäkalien fressen.

Die Operation erfolgte unter Äthernarkose. Danach wurde der Pylorus abgebunden, so daß jede Verbindung zwischen Magen und Duodenum unterbrochen war. Zum Abstoppen der eigenen Magensaftproduktion wurden subkutan 2 mg Atropin/kg oder 10 mg Cimetidin/kg Ratte gleich nach Abbinden der Pylorus allen Gruppen gegeben.

Unmittelbar anschließend wurde das erfindungsgemäße pharmazeutische Mittel in der aus der folgenden Tabelle ersichtlichen Form bzw. Konzentration gegeben und zwar in einer Lösung in destilliertem Wasser in einer Menge von jeweils 3 mg Lysinorotat bzw. Inosin oder 1 mg Thymin pro ml, wobei pro Ratte von 1,5 ml gegeben wurde. An Vitamin A wurde 375 i. E. pro Ratte gegeben. Die Zugabe erfolgte durch eine Sonde oder eine Kanüle. Eine Stunde später wurde dann 0,5 normale Salzsäure plus 1% Pepsin gegeben und zwar in einer Menge von 2 ml pro Ratte. Fünf Stunden später wurden die Tiere getötet und sodann die Magen untersucht. Die Testkombination, Konzentration und Ergebnisse sind aus der folgenden Tabelle zu entnehmen. Die Beurteilung der gewonnenen Ergebnisse wurden durch 3 Personen durchgeführt, die nicht informiert wurden, um welche Testgruppen es sich handelte. Es ist vereinbart, daß der Unterschied von 1 Note zwischen zwei Gruppen den Ulzerationsunterschied von annähernd etwa 50% bedeutet. Das heißt, daß eine Gruppe die mit Note 4 beurteilt ist, Ulzera doppelt so stark entwickelt, als die Gruppe, die mit Note 2 beurteilt ist. Die Note 1 wird nur in dem Falle gegeben, wo keine Ulzera vorliegen. Bei dieser Benotung sind jeweils die Vegleichswerte gegeneinander zu stellen (Komparationen), da es keine absolut gültigen Werte gibt.

Tabelle

Darstellung der Tierversuche an 580 Shay-Ratten
Heilung von künstlich hervorgerufenen Magengeschwüren

Beste Note = 1 d. h. bei Note 1 überhaupt kein Ulcus mehr
Die Noten gehen von 1 bis 5

|  | Note Mittelwert | |
|---|---|---|
| Lysin-Orotat | 2,8 | |
| Inosin | 3,3 | |
| Lysinorotat + Inosin | 2,1 | Kontrollversuch 76% schlechter |
| Kontrollversuch | 3,7 | |
| Lysin-Orotat | 2,9 | d. h. Lysin-Orotat um etwa 25% schlechter als die Kombination |
| Lysin-Orotat + Inosin | 2,4 | Lysin-Orotat + Inosin |
| Lysin-Orotat | 3,0 | Lysin-Orotat ist im Vergleich mit der Kombination Lysin-Orotat + Thymin um |
| Lysin-Orotat + Thymin | 1,9 | 50% schlechter |
| Kontrollversuch | 3,8 | Kontrollversuch 115% schlechter |
| Lysin-Orotat + Inosin + Thymin | 1,5 | |
| Lysin-Orotat + Inosin | 2,5 | Lysin-Orotat + Inosin ist gegenüber der 3er Kombination um 24% schlechter |
| Lysin-Orotat + Inosin + Thymin | 2,1 | |
| Lysin-Orotat + Inosin + Vitamin A | 1,3 | Lysin-Orotat + Inosin ist gegenüber der Kombination |
| Lysin-Orotat + Inosin | 2,6 | Lysin-Orotat + Inosin + Vitamin A um 100% schlechter |

Beim Menschen wird das erfindungsgemäße pharmazeutische Mittel meist in wäßriger Lösung gegeben.

Es wurden Patienten ausgewählt, die bezüglich der vorgenannten Krankheiten eine langjährige Erfahrung in der Anwendung anderer an sich bekannter Medikamente besitzen z. B. von Cimetidin, Biogastrone, Antiacida und Sedativa.

Die Patienten bzw. Probanden mit Gastritis oder Magengeschwüren erhielten zweimal täglich 25 mg je Wirkstoff (Lysinorotat$_2$ + Inosin) + Vitamin B$_{12}$ + Folsäure des erfindungsgemäßen Mittels (unverdünnt gerechnet) pro Tag. Meistens wurden die pharmazeutischen Mittel in einem Tee oder in verdünnter Milch gegeben.

Generell läßt sich sagen, daß bereits nach 3 bis 7 Tagen die Schmerzen abgeklungen waren und keine Komplikationen aufgetreten sind.

Insgesamt wurden 36 Probanden auf diese Weise behandelt.

Lediglich bei zwei Probanden kamen im Frühling wieder die alten Beschwerden, jedoch nur in schwächerer Form.

Nach Wiedereinnahme der erfindungsgemäßen pharmazeutischen Mittel innerhalb von zwei bis drei Tagen in der oben genannten Quantität, also zweimal pro Tag 25 mg je Wirkstoff (Lysinorotat + Inosin) + Vitamin sind die Schmerzen wieder abgeklungen.

Bei festgestellten Magengeschwüren (12 Patienten) wurde die Behandlung 3 Wochen lang durchgeführt. Es gab nur einen Resistentfall.

Bei Paradentose wurden 28 Probanden behandelt, wobei es sich um akute Paradentose handelte. Die Patienten bzw. Probanden litten an Blutungen des Zahnfleisches beim Zähneputzen mit cyanotischer Färbung des Zahnfleisches und unangenehmem Gefühl im Zahnfleisch.

Sie erhielten folgende Dosen:

2 mg je Wirkstoff (Lysinorotat + Inosin) + Vitamin A + $B_{12}$ + Folsäure pro ml wässeriger Lösung des erfindungsgemäßen pharmazeutischen Mittels.

Mit dieser wäßrigen Lösung wurde das Zahnfleisch zweimal täglich einmassiert. Die Dauer des Einmassierens betrug jeweils etwa 20 Minuten.

Bereits nach 48 Stunden hörten die Blutungen auf. Nach 4 Tagen der vorgenannten Massage war auch die blaue cyanotische Färbung des Zahnfleisches verschwunden, der Heileffekt war bei allen Patienten sichtbar. Bei 10 anderen Patienten wurden Beschädigungen an den Lippen des Mundes, sogenannten Fissuren und Raghaden, die äußerst widerstandsfähig waren, dadurch behandelt und geheilt, daß die Lippen zweimal am Tag eine halbe Stunde lang mit Watte mit dem pharmazeutischen Mittel in der oben genannten Konzentration angefeuchtet wurden.

Auch hier war der pathologische Zustand nach 24 bis 48 Stunden verschwunden, d. h. es war Heilung eingetreten.

Bei Stomatitis aphtósa wurden 18 Patienten behandelt, die an rezidivierenden Aphten litten.

Erfindungsgemäßes pharmazeutisches Mittel wurde in folgenden Dosen angewendet: 3 mg je Wirkstoff pro ml, (Lysinorotat + Inosin).

Die Patienten wurden so behandelt, daß die Ulcera zweimal täglich 20 Minuten mit Watte, die mit dem pharmazeutischen Mittel in obiger Konzentration angefeuchtet war, leicht gedrückt wurden.

Eine Verbesserung und Erleichterung tritt binnen 24 bis 48 Stunden ein. Die Abheilung tritt nach 3 bis 5 Tagen ein.

Eine systematische Behandlung mit dem Mittel 1 bis 2mal wöchentlich in einer Dauer von 15 Minuten (es reicht, 2—3 ml des Mittels nur im Mund zu halten und leicht zu bewegen) hat einem Rezidiv der Krankheit völlig vorgebeugt. Der Erfolg erschien in allen Fällen.

Ferner wurden 14 Patienten, die an sehr schwerer chronischer Konjunktivitis litten, die gegen andere Medikamente des Standes der Technik sich als sehr widerstandsfähig herausgestellt hat, mit dem erfindungsgemäßen pharmazeutischen Mittel behandelt.

Es wurde wieder die obige Konzentration, nämlich eine wäßrige Lösung von 1 mg je Wirkstoff (Lysinorotat + Inosin) + Vitamin $B_{12}$ + Folsäure pro ml in destilliertem Wasser gelöst und nur 1 Tropfen vor dem Schlafengehen mit einer Gewährleistung einer 15minütigen Einwirkungszeit durch dauerndes 15minütiges Schließen der Augen angewendet. Nach einer Behandlungszeit von 6 bis 14 Tagen haben die Probanden keinerlei Anzeichen von Konjunktivitis mehr. Der Heilerfolg bei der Konjunktivitis ist leicht daran zu erkennen, daß das bekannte Gefühl der Patienten, sie hätten Sandkörnchen im Auge, nach der Behandlung mit dem erfindungsgemäßen Mittel verschwunden war. 11 von 14 Patienten waren nach 7 bis 14 Tagen völlig geheilt.

Die Dreierkombination (Lysinorotat, Inosin, Thymin) wurde bei etwa 30% der Patienten gegeben. Ein leichter Vorteil dieser Kombination gegenüber der Zweierkombination ist in etwa festzustellen.

Bei den weiter oben genannten Tierversuchen war Vitamin $B_{12}$ und Folsäure nicht vorhanden.

Die Konzentration an »Vitamin« war die folgende:

— Vitamin A (wasserlösliche Form) 250 i. E./ml oder Gramm
— $B_{12}$ 0,05 mg/ml oder Gramm
— Folsäure 0,05 mg/ml oder Gramm
— Thymin wurde in allen Untersuchungen in einer Menge von 1/4 der Lysinorotat-Dosis gegeben.

Wegen der hier verwendeten chemischen Nomenklatur wird vorsorglich auf das Standardwerk: DNA Synthese von Arthur Kornberg (Stanford University) W. H. Freemann und Company-Verlag, San Francisco hingewiesen.

Das erfindungsgemäße pharmazeutische Mittel liegt vorzugsweise in wasserlöslicher Form, in Tablettenform oder in Kapselform vor, und wird vorzugsweise in dieser Form appliziert.

## Patentansprüche

1. Pharmazeutisches Mittel zur Heilung von entzündlichen und/oder degenerativen und/oder atrophischen Schleimhauterkrankungen, gekennzeichnet durch einen Gehalt an:

a) dem Nukleosid Inosin
b) dem Lysinorotat.

2. Pharmazeutisches Mittel nach Anspruch 1, dadurch gekennzeichnet, daß es Vitamin A und/oder Vitamin $B_{12}$ und/oder Folsäure enthält.

3. Pharmazeutisches Mittel nach Anspruch 1 oder 2, dadurch gekennzeichnet, daß es Vitamin A und/oder Thymin enthält.

4. Pharmazeutisches Mittel nach mindestens einem der Ansprüche 1 bis 3, dadurch gekennzeichnet, daß es mindestens einen an sich bekannten Salbengrundstoff enthält.

5. Pharmazeutisches Mittel nach Anspruch 4, dadurch gekennzeichnet, daß es mindestens einen an sich bekannten Hautsalbengrundstoff enthält.

6. Pharmazeutisches Mittel nach mindestens einem der Ansprüche 1 bis 5, dadurch gekennzeichnet, daß es in Gelform vorliegt.

7. Pharmazeutisches Mittel nach mindestens einem der Ansprüche 1 bis 6, dadurch gekennzeichnet, daß es·in wasserlöslicher Form vorliegt.

8. Pharmazeutisches Mittel nach mindestens einem der Ansprüche 1 bis 3 und 7, dadurch gekennzeichnet, daß es als Tablette vorliegt.

9. Pharmazeutisches Mittel nach mindestens einem der Ansprüche 1 bis 3 und 7, dadurch gekennzeichnet, daß es in Kapselform vorliegt.


## Claims

1. Pharmacentical agent for healing of inflammatory and/or degenerative and/or atrophic mucous membrance diseases, thus characterized by a content of

a)   the nucleoside inosin
b)   the lysine orotate.

2. Pharmacentical agent according to claim 1, thus characterized that it contains vitamin A and/or vitamin $B_{12}$ and/or folic acid.

3. Pharmacentical agent according to claim 1 or 2, thus characterized that it contains vitamin A and/or thymine.

4. Pharmacentical agent according to at least one of the claims 1 till 3, thus characterized that it contains at least one ointment base, known as such.

5. Pharmacentical agent according to claim 4, thus characterized that it contains at least one skin ointment base, known as such.

6. Pharmacentical agent according to at least one of the claims 1 till 5, thus characterized that it exists in form of a gel.

7. Pharmacentical agent according to at least one of the claims 1 till 6, thus characterized that it exists in water-soluble form.

8. Pharmacentical agent according to at least one of the claims 1 till 3 and 7, thus characterized that it exists as tablet.

9. Pharmacentical agent according to at least one of the claims 1 till 3 and 7, thus characterized that it exists in form of a capsule.


## Revendications

1. Moyen pharmaceutique pour la guérison des maladies de la membrane mugeuse inflammatoire et/ou dégénerative et/ou atrophique, caractérisé en ce qu'il contient

a)   le nucléoside inosine
b)   l'orotate de lysine.

2. Moyen pharmaceutique selon revendication 1, caractérisé en ce qu'il contient vitamine A et/ou vitamine $B_{12}$ et/ou acide folique.

3. Moyen pharmaceutique selon revendication 1 ou 2, caractérisé en ce qu'il contient vitamine A et/ou thymine.

4. Moyen pharmaceutique selon au moins l'un revendications 1 à 3, caracterisé en ce qu'il contient au mois une base de onguent connue en elle-même.

5. Moyen pharmaceutique selon revendication 4, caractérisé en ce qu'il contient au moins une base de onguent pour la peau connue en elle-même.

6. Moyen pharmaceutique selon au moins l'une des revendications 1 à 5, caractérisé en ce qu'il y a dans une forme de gel.

7. Moyen pharmaceutique selon au moins l'une des revendications 1 à 6, caractérisé en ce qu'il y a dans une forme soluble dans l'eau.

8. Moyen pharmaceutique selon au moins l'une des revendications 1 à 3 et 7, caractérisé en ce qu'il a comme comprimé.

9. Moyen pharmaceutique selon au moins l'une des revendications 1 à 3 et 7, caractérisé en ce qu'il y a dans une forme de capsule.